# EUROPEAN PATENT APPLICATION

(11) **EP 3 530 354 A1**
(43) Date of publication of application: **28.08.2019**
(21) Application number: 17865582.5
(22) Date of filing: 20.10.2017
(51) Int. Cl.: B01L 3/14

(54) **COMBINATION-TYPE CENTRIFUGAL DEVICE**

(30) Priority: 24.10.2016 CN 201610936523
(71) Applicant: Shenzhen Peacock Biotechnology Co., Ltd, Shenzhen Guangdong 518000 (CN)
(72) Inventor: YOU, Jianqin, Shenzhen Guangdong 518000 (CN)
(74) Representative: Keil & Schaafhausen Patent- und Rechtsanwälte PartGmbB
(86) International application number: PCT/CN2017/107091
(87) International publication number: WO 2018/077120

(57) **Abstract**

The present invention discloses a combined centrifugal device, including: a tubule; and a negative pressure storage chamber configured to generate a negative pressure and store a suspension, wherein the negative pressure storage chamber is in communication with the tubule so that the negative pressure storage chamber generates a negative pressure to suck the suspension into the negative pressure storage chamber and the tubule through the tubule; wherein the tubule is provided with a fixing means through which the combined centrifugal device is fixed to the centrifugal machine. In such a way, the present invention fixes the combined centrifugal device to the centrifugal machine by means of the fixing means, so that the centrifugal machine can use the tubule to store the rare content of ingredients in the suspension during centrifugation to directly enlarge the thickness of the rare content of ingredients in the suspension in the tubule, therefore, the rare content of ingredients in the suspension is effectively thickened and stratified.

## Description

### Field of the Invention

The present invention relates to the field of medical technology, in particular to a combined centrifugal device applicable to stratifying cellular ingredients.

### Background of the Invention

The centrifugal technology is widely applied to cell biology, molecular biology and biochemistry and other related fields. Under the action of centrifugal force, different proportions of cells in the suspension are stratified and then separated and concentrated.

The traditional centrifugal tube is a perfusion-type tube, that is, a suspension to be stratified is injected into the centrifugal tube from top to bottom, and the upper diameter of the central tube is greater than or equal to the middle and bottom diameters.

In the traditional centrifugation operation, cells of different specific gravities in the suspension stay at different levels of the centrifugal tube after centrifugation, and the layer formed by the rare content of some cellular ingredients in the traditional centrifugal tube is very thin and difficult to suck and separate. Taking peripheral blood as an example, anti-coagulated peripheral blood is composed of plasma and blood cells which mainly include red blood cells, granulocytes and mononuclear cells. Because of different proportions, blood cells will be distributed at different levels of the centrifugal tube after natural sedimentation or centrifugation. Mononuclear cells in the peripheral blood account for only about one-thousandth of ingredients of the blood cells. After centrifugation, the red blood cells and the granulocytes are located in the lower layer of the centrifugal tube, the plasma is located in the upper layer of the central tube, and the mononuclear cells form a thin layer in the middle layer of the central tube, and are very difficult to suck because of the rare content.

### Summary of the Invention

The technical problem to mainly be solved by the present invention is to provide a combined centrifugal device capable of effectively separating the rare content of ingredients in the suspension.

In order to solve the technical the following technical solution is adopted in the present invention: a combined centrifugal device is provided, including: a tubule; and a negative pressure storage chamber configured to generate a negative pressure and store a suspension, wherein the negative pressure storage chamber is in communication with the tubule so that the negative pressure storage chamber generates a negative pressure to suck the suspension into the negative pressure storage chamber and the tubule through the tubule; wherein the tubule is provided with a fixing means through which the combined centrifugal device is fixed to a centrifugal machine, so that the suspension in the combined centrifugal device is centrifuged through the centrifugal machine.

The fixing means is a snap-fit mechanism, which is configured to be stuck on the centrifugal machine to fix the combined centrifugal device to the centrifugal machine. The fixing means is a centrifugal cover connected to the central tube, and the tubule is inserted into the central cover so as to fix the combined centrifugal device in the centrifugal tube.

The negative pressure storage chamber includes an elastic container to generate a negative pressure when the elastic container is squeezed.

The negative pressure storage chamber includes at least two intercommunicated elastic containers to generate a negative pressure when one of the at least two intercommunicated elastic containers is squeezed.

The combined centrifugal device further includes a clamping component, configured to be clamped between the at least two intercommunicated elastic containers.

The clamping component includes a pinch valve.

The negative pressure storage chamber is provided with an opening, configured to be connected with a negative pressure generator to generate a negative pressure by a negative pressure generator, wherein the negative pressure generator is an injection-type syringe or a vacuum pump.

The inner diameter of the tubule is less than or equal to 2 mm, and the width cross-sectional area of the negative pressure storage chamber is 3-20 times the width cross-sectional area of the tubule.

The tubule is transparent or translucent, the negative pressure storage chamber is transparent or translucent, and the tubule is integrally formed with the negative pressure storage chamber.

The present invention has the beneficial effects that different from the condition of the prior art, the combined centrifugal device disclosed by the present invention includes the tubule; and the negative pressure storage chamber configured to generate a negative pressure and store a suspension, wherein the negative pressure storage chamber is in communication with the tubule so that the negative pressure storage chamber generates a negative pressure to suck the suspension into the negative pressure storage chamber and the tubule through the tubule; wherein the tubule is provided with the fixing means through which the combined centrifugal device is fixed to the centrifugal machine. In such a way, the present invention can directly fix the combined centrifugal device to the centrifugal machine by means of the fixing means, so that the centrifugal machine can use the tubule to store the rare content of ingredients in the suspension during centrifugation to directly enlarge the thickness of the rare content of ingredients in the suspension in the tubule, which facilitates better separating out the rare content of ingredients in the suspension, therefore, the efficiency is greatly improved and the stratifying and separation effects are better.

### Brief Descriptions of the Drawings

Fig.1 is a schematic view of a first structure of a combined centrifugal device according to the present invention;
Fig.2 is a schematic view showing a connection structure of the combined centrifugal device according to the present invention and an injection-type syringe;
Fig.3 is a schematic view of a second structure of the combined centrifugal device according to the present invention;
Fig.4 is a schematic view of a third structure of the combined centrifugal device according to the present invention;
Fig.5 is a schematic view showing the connection structure of the combined centrifugal device according to the present invention and a centrifugal tube.

### Detailed Description of the Embodiments

The present invention will now be described in detail in conjunction with the drawings and embodiments.

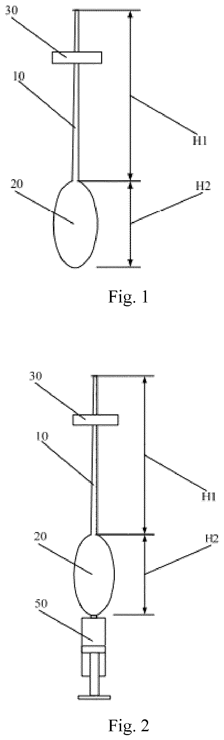

As shown in Fig.1, Fig.1 is a schematic view of a first structure of a combined centrifugal device according to the present invention. The combined centrifugal device includes a tubule 10, a negative pressure storage chamber 20, and a fixing means 30.

The tubule 10 is configured to suck a suspension. It should be understood that the suspension may specifically be blood, peripheral blood or other body mixed suspension components.

In the present embodiment, the inner diameter of the tubule 10 is less than or equal to 2 mm, and due to such a fact, rare ingredients are thickened in the tubule, which is more advantageous for stratifying and separation. In addition, as particles are present in the suspension and the suspension has a certain viscosity, when the inner diameter of the tubule 10 is less than or equal to 2 mm, the suspension will stay in the tubule 10 without any pressure, so that the suspension seals the tubule 10.

The negative pressure storage chamber 20 is configured to store a suspension, wherein the negative pressure storage chamber 20 is in communication with the tubule 10. In the present embodiment, the negative pressure storage chamber 20 can generate a negative pressure such that when the negative pressure storage chamber 20 generates a negative pressure, the suspension is sucked into the negative pressure storage chamber 20 and/or the tubule 10 through the tubule 10.

In the present embodiment, the negative pressure storage chamber 20 includes an elastic container to generate a negative pressure when the elastic container 20 is squeezed. It should be understood that the present invention does not limit the shape of the elastic container, and the container elastic may specifically be of a spherical, cylindrical, rectangular, elliptical or other irregular shape.

Specifically, the negative pressure storage chamber 20 includes an elastic container made of an elastic material, that is, the elastic container is restored to its original shape when squeezed. When in use, the tubule 10 is placed in the suspension, and a user squeezes the elastic container by hands to discharge residual air inside the elastic container at first, so that a negative pressure is formed inside the elastic container to suck the suspension into the elastic container and/or the tubule 10 through the tubule 10. In addition, after the tubule 10 sucks up the suspension, a small amount of bubbles will occur. Since the negative pressure storage chamber 20 is an elastic container, the residual bubbles can be completely discharged by repeatedly shaking and squeezing the elastic container and sucking the suspension.

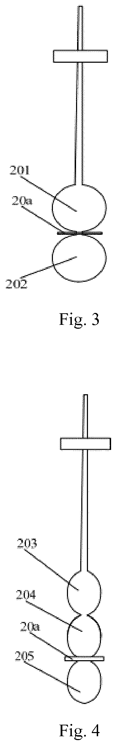

Of course, in some embodiments, the negative pressure storage chamber 20 includes at least two intercommunicated elastic containers to generate a negative pressure when one of the at least two intercommunicated elastic containers is squeezed. The combined centrifugal device further includes a clamping component 20a, configured to be clamped between the at least two intercommunicated elastic containers, wherein the clamping component 20a may specifically include a pinch valve. Of course, the embodiment does not limit that the clamping component 20a includes the pinch valve. In other embodiments, the clamping component 20a may also include other clamping components. It should be understood that the present invention does not limit the shape and number of the at least two intercommunicated elastic containers. Specifically, as shown in FIG. 3, at least two intercommunicated elastic containers include a first spherical elastic container 201 and a second spherical elastic container 202 connected thereto, and the clamping component 20a is clamped between the first spherical elastic container 201 and the second spherical elastic container 202. Of course, the first spherical elastic container 201 and the second spherical elastic container 202 are connected by a small tubule, and the clamping component 20a is clamped on the small tubule. Alternatively, as shown in FIG. 4, the at least two intercommunicated elastic containers include a first elliptical elastic container 203, a second elliptical elastic container 204 in communication with the first elliptical elastic container 203, and a third elliptical elastic container 205 in communication with the second elliptical elastic container 204. The clamping component 20a is clamped among the first elliptical elastic container 203, the second elliptical elastic container 204, and the third elliptical elastic container 205. Specifically, the clamping component 20a is clamped between the elliptical elastic container 204 and the third elliptical elastic container 205. Of course, the first elliptical elastic container 203, the second elliptical elastic container 204, and the third elliptical elastic container 205 are in communication through a small tubule, and the clamping component 20a is clamped on the small tubule.

Specifically, the at least two intercommunicated elastic containers are each an elastic container made of an elastic material, that is, a negative pressure is generated when the containers are squeezed. When in use, the tubule 10 is placed in the suspension, and a user squeezes the one of the at least two intercommunicated elastic containers by hands to discharge residual air inside the at least two intercommunicated elastic containers at first, so that a negative pressure is formed inside the at least two intercommunicated elastic containers to suck the suspension into the at least two intercommunicated elastic containers through the tubule 10. In addition, it should be noted that a small amount of air remains inside the negative pressure storage chamber 20 when the suspension is sucked through the tubule 10, so that by providing at least two intercommunicated elastic containers and clamping the clamping component 20a between two intercommunicated elastic containers, the remaining small amount of air can be isolated in one of the at least two intercommunicated elastic containers (i.e., the one farthest from the tubule 10), and all of the rest of the at least two intercommunicated elastic containers can be used to store the suspension to ensure that the suspension has no bubbles. In this embodiment, it can be convenient and quick to isolate the bubbles at the tail or other locations of the elastic container by means of more than two elastic containers and the clamping component 20a. Of course, it is also possible to suck the suspension in combination with the way of shaking and repeatedly squeezing the elastic container multiple times to further discharge the residual bubbles, and discharging the bubbles in multiple ways ensures that the suspension in the negative pressure storage chamber 20 has no bubble.

Of course, in some embodiments, as shown in FIG. 2, the negative pressure storage chamber 20 is provided with an opening, configured to be connected with the negative pressure generator to generate a negative pressure by the negative pressure generator. It should be understood that the present embodiment does not limit the position where the negative pressure storage chamber 20 is provided with an opening, and the opening may be disposed at the bottom or side or other position of the negative pressure storage chamber 20. The negative pressure generator may specifically be an injection-type syringe 50 or a vacuum pump to generate a negative pressure by the injection-type syringe 50 or the vacuum pump. It should be noted that the inner diameter of the opening is less than or equal to 2 mm, in such a way that the suspension is sucked into a rigid container through the injection-type syringe 50 or the vacuum pump. Of course, the negative pressure storage chamber 20 may be further provided with a sealing cover thereon for sealing the opening. More specifically, the negative pressure storage chamber 20 may actually be a rigid container having an opening for connection with the negative pressure generator to generate a negative pressure by the negative pressure generator, and the rigid container may be further provided with a sealing cover for sealing the opening.

In the present embodiment, the tubule 10 is transparent or translucent, that is, the tubule 10 is made of a transparent or translucent material, so that the condition of the suspension in the tubule 10 can be seen, i.e., the thickness of the rare components in the suspension in the tubule 10. Of course, the tubule 10 can also be provided with scales through which the thickness of the suspension in the tubule 10 can be read.

In the present embodiment, the negative pressure storage chamber 20 is transparent or translucent, that is, the negative pressure storage chamber 20 is made of a transparent or translucent material, so that the condition of the suspension in the negative pressure storage chamber 20 can be seen, i.e., the thickness of the rare components in the suspension in the negative pressure storage chamber 20. Of course, the negative pressure storage chamber 20 can also be provided with scales through which the thickness of the suspension in the negative pressure storage chamber 20 can be read. In the present embodiment, the tubule 10 and the negative pressure storage chamber 20 are integrally formed, so that the structure is simple, and the design cost can be reduced. Of course, in other embodiments, the tubule 10 is detachably disposed on the negative pressure storage chamber 20. Specifically, the tubule 10 is in threaded connection with the negative pressure storage chamber 20, that is, the tubule 10 is provided with external threads, and the negative pressure storage chamber 20 is provided with internal threads, the external threads are connected with the internal threads, so that detachable connection between the tubule 10 and the negative pressure storage chamber 20 can be achieved.

In this embodiment, the inner diameter of the tubules 10 is unchanged. Of course, in other embodiments, the inner diameter of the tubule 10 decreases gradually, that is, the inner diameter of the tubule 10 gradually decreases from the end connected to the negative pressure storage chamber 20 to the other end away from the negative pressure storage chamber 20, so that the tiny rare contents can be amplified in tubule 10.

In the present embodiment, the inner diameter of the tubule 10 is in cambered connection with the inner diameter of the negative pressure storage chamber 20, so that the rare content of ingredients in the suspension can be quickly separated into the tubule 10, preferably the angle of the cambered connection at the junction of the tubule 10 and the negative pressure storage chamber 20 is 30-60 degrees.

In addition, the fixing means 30 is disposed on the tubule 10 to fix the combined centrifugal device to the centrifugal machine by the fixing means 30, so that the suspension in the combined centrifugal device is centrifuged by the centrifugal machine, that is, the rare content of ingredients in the suspension in the negative pressure storage chamber 20 can be separated in the tubule 10 by the centrifugal machine. The suspension is directly centrifuged through the combined centrifugal device with a simple structure, thus greatly reducing the operation time and the design cost.

In the present embodiment, the inner diameter of the negative pressure storage chamber 20 is unchanged, and the width cross-sectional area of the negative pressure storage chamber 20 is 3-20 times the width cross-sectional area of the tubule 10, specifically, the width cross-sectional area of the negative pressure storage chamber 20 is 10 times the width cross-sectional area of the tubule 10 (that is, in the case where the inner diameter of the tubule 10 is unchanged). Therefore, as long as the suspension of the negative pressure storage chamber 20 contains the rare content of ingredients, the ingredients can be enlarged and displayed in the tubule 10, in such a way, the ingredients in the tubule 10 can be directly sucked out or the tubule 10 is cut away and then the ingredients in the tubule 10 are taken out, so that the rare content of ingredients in the suspension can be separated out more conveniently. Of course, the length H1 of the tubule 10 is 10-20 times the length of the negative pressure storage chamber 20. Of course, in other embodiments, the inner diameter of the negative pressure storage chamber 20 may also be changed, that is, the negative pressure storage chamber 20 is of a spherical shape, a gourd shape or an irregular shape.

In the present embodiment, the fixing means 30 and the tubule 10 are integrally formed. Of course, in other embodiments, the fixing means 30 is detachably disposed on the tubule 10.

In the present embodiment, the fixing means 30 is a snap-fit mechanism, which is configured to be stuck on the centrifugal machine to fix the combined centrifugal device to the centrifugal machine. Specifically, the snap-fit mechanism 30 is fixed in the tubule 10, and is provided with a protrusion, and the panel or plug of the centrifugal machine is provided with a groove, and the protrusion is stuck in the groove and in clearance fit with the groove, so that the combined centrifugal device is stuck on the centrifugal machine.

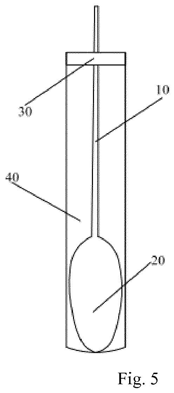

In some embodiments, as shown in FIG. 5, the fixing means 30 is a centrifugal cover connected to the centrifugal tube 40, and the tubule 10 is inserted in the central cover to fix the combined centrifugal device in the centrifugal tube 40, so that the combined centrifugal device is fixed to the centrifugal machine by the centrifugal tube 40.

It should be understood that the tubule 10 is detachably disposed in the central cover. Specifically, the central cover is provided with an opening into which the tubule 10 is inserted. In addition, the outer diameter of the tubule 10 gradually decreases from the end connected to the negative pressure storage chamber 20 to the other end away from the negative pressure storage chamber 20. When the outer diameter of the tubule 10 is equal to the inner diameter of the opening, the centrifugal cover card is stuck in the tubule 10 at this time, and the tubule 10 can be fixed in the central tube by means of the centrifugal cover. Of course, in other embodiments, the tubule 10 and the centrifugal cover are integrally formed.

It should be understood that the tubule 10 and the negative pressure storage chamber 20 are longer than the centrifugal tube 40, that is, when the combined centrifugal device is fixed in the centrifugal tube 40, the negative pressure storage chamber 20 is disposed at the bottom of the centrifugal tube 40 to support the negative pressure storage chamber 20 through the bottom of the centrifugal tube 40, such that the negative pressure storage chamber 20 does not move during centrifugation. In addition, when the combined centrifugal device is fixed in the centrifugal tube 40, the end of the tubule 10 is higher than the level height of the fixing means 30, so that even when the tubule 10 properly displace under the centrifugal effect during centrifugation, and the stratifying and thickening effects are not affected.

In this embodiment, the mixed suspension is sucked in a manner of generating a negative pressure, thereby avoiding the operation way in the conventional technology and the prior art that the suspension is poured into the centrifugal tube to reduce pollution and generated bubbles. Meanwhile, in the present invention, as the combined centrifugal device is provided with a slight tubule-type structure, in the case of the same volume of suspension, especially a small volume of or tiny suspension, the tubule of the combined centrifugal device can well enlarge the thickness of the rare content of ingredients in the suspension in the negative pressure storage chamber after centrifugation of the centrifugal machine, which facilitates better separating out the rare content of cellular ingredients through centrifugation.

In summary, the combined centrifugal device disclosed by the present invention includes: the tubule; and the negative pressure storage chamber configured to generate a negative pressure and store a suspension, wherein the negative pressure storage chamber is in communication with the tubule so that the negative pressure storage chamber generates a negative pressure to suck the suspension into the negative pressure storage chamber and the tubule through the tubule; wherein the tubule is provided with the fixing means through which the combined centrifugal device is fixed to the centrifugal machine. In such a way, the present invention can directly fix the combined centrifugal device to the centrifugal machine by means of the fixing means, so that the centrifugal machine can use the tubule to store the rare content of ingredients in the suspension during centrifugation to directly enlarge the thickness of the rare content of ingredients in the suspension in the tubule, which facilitates better separating out the rare content of ingredients in the suspension, therefore, the efficiency is greatly improved and the stratifying and separation effects are better. The above description is only the embodiments of the present invention, and is not intended to limit the patent scope of the present invention. Any equivalent structures or equivalent process transformations made by using the description and the drawings of the present invention, directly or indirectly applied to other related technical fields, are all encompassed in the scope of patent protection of the present invention in a similar way.

## Claims

1. A combined centrifugal device, comprising:
a tubule;
a negative pressure storage chamber configured to generate a negative pressure and
store a suspension, wherein the negative pressure storage chamber is in communication with the tubule so that the negative pressure storage chamber generates a negative pressure to suck the suspension into the negative pressure storage chamber and the tubule through the tubule;
wherein the tubule is provided with the fixing means through which the combined centrifugal device is fixed to a centrifugal machine, so that the suspension in the combined centrifugal device is centrifuged through the centrifugal machine.

2. The combined centrifugal device according to claim 1, **characterized in that** the fixing means is a snap-fit mechanism, which is configured to be stuck on the centrifugal machine to fix the combined centrifugal device to the centrifugal machine.

3. The combined centrifugal device according to claim 1, **characterized in that** the fixing means is a centrifugal cover connected to the central tube, and the tubule is inserted into the central cover so as to fix the combined centrifugal device in the centrifugal tube.

4. The combined centrifugal device according to claim 1, **characterized in that** the negative pressure storage chamber comprises an elastic container to generate a negative pressure when the elastic container is squeezed.

5. The combined centrifugal device according to claim 4, **characterized in that** the negative pressure storage chamber comprises at least two intercommunicated elastic containers to generate a negative pressure when one of the at least two intercommunicated elastic containers is squeezed.

6. The combined centrifugal device according to claim 5, **characterized in that** the combined centrifugal device further comprises a clamping component, configured to be clamped between the at least two intercommunicated elastic containers.

7. The combined centrifugal device according to claim 6, **characterized in that** the clamping component comprises a pinch valve.

8. The combined centrifugal device according to claim 1, **characterized in that** the negative pressure storage chamber is provided with an opening, configured to be connected with the negative pressure generator to generate a negative pressure by the negative pressure generator, wherein the negative pressure generator is an injection-type syringe or a vacuum pump.

9. The combined centrifugal device according to claim 1, **characterized in that** the inner diameter of the tubule is less than or equal to 2 mm, and the width cross-sectional area of the negative pressure storage chamber is 3-20 times the width cross-sectional area of the tubule.

10. The combined centrifugal device according to claim 1, **characterized in that** the tubule is transparent or translucent, the negative pressure storage chamber is transparent or translucent, and the tubule is integrally formed with the negative pressure storage chamber.
